(19) **Europäisches Patentamt European Patent Office Office européen des brevets**

(11) **EP 4 655 278 B1**

(12) **EUROPEAN PATENT SPECIFICATION**

(45) Date of publication and mention
of the grant of the patent:
**20.05.2026 Bulletin 2026/21**

(51) International Patent Classification (IPC):
***C07C 51/00*** (2006.01)   ***C07C 55/10*** (2006.01)
***C07C 59/185*** (2006.01)

(21) Application number: **25706804.9**

(22) Date of filing: **21.02.2025**

(52) Cooperative Patent Classification (CPC):
(C-Sets available)
**C07C 51/00**                         (Cont.)

(86) International application number:
**PCT/EP2025/054733**

(87) International publication number:
**WO 2025/176848 (28.08.2025 Gazette 2025/35)**

(54) **PRODUCTION OF SUCCINIC ACID FROM WOOD WASTE**

HERSTELLUNG VON BERNSTEINSÄURE AUS HOLZABFALL

PRODUCTION D'ACIDE SUCCINIQUE À PARTIR DE DÉCHETS DE BOIS

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **22.02.2024 EP 24159247**

(43) Date of publication of application:
**03.12.2025 Bulletin 2025/49**

(73) Proprietor: **Technische Hochschule Rosenheim
83024 Rosenheim (DE)**

(72) Inventors:
• **ZENZ, Vitus
83024 Rosenheim (DE)**

• **STRÜBBE, Nicole
83052 Bruckmühl (DE)**
• **MUSCAT, Dirk
85521 Ottobrunn (DE)**
• **KREY, Adrian
83539 Pfaffing (DE)**

(74) Representative: **Hoefer & Partner Patentanwälte
mbB
Pilgersheimer Straße 20
81543 München (DE)**

(56) References cited:
**CN-B- 113 149 822      US-A1- 2019 382 328**

(52) Cooperative Patent Classification (CPC): (Cont.)

C-Sets
**C07C 51/00, C07C 55/10;**
**C07C 51/00, C07C 59/185**

## Description

### Technical field

The invention refers a chemical method for the continuous production of succinic and levulinic acid, in particular succinic acid, at high yields from biomass in a continuous flow reaction pipe.

### Background art

Current biomass based succinic and levulinic acid production faces the challenge of high raw material prices, an overall low yield and the production of low-value by-products. This has impaired the competitiveness of biomass based production processes to date. Biomass based succinic acid and levulinic acid are still not economically viable compared to petrochemically produced succinic acid and levulinic acid. In addition to the long production time of fermentation processes, the reasons include the separation of the resulting sludge (residual materials). Even if high yields can be achieved with microorganisms, the complex and energy-intensive separation process significantly reduces the profitability of this method. Taken together, these challenges make a microorganism-based approach to succinic acid production difficult for large-scale biorefineries.

Moreover, microorganism-based production processes use batch processes to produce succinic and levulinic acid. These batch fermentation processes have the disadvantage of high investment and operating costs. Large-scale plants favour the development of continuous processes whenever possible, as they offer higher productivity due to less downtime, e.g. for the necessary cleaning before the next batch process. In addition, the high development costs and customised production of special batch reactors lead to considerable initial investment.

US5143834A describes a fermentation batch process to produce succinic acid from carbohydrates, sodium ions and tryptophan under pressure and with the aid of microorganisms. The product must then be separated from sulphate ions and sulphuric acid in order to obtain high-purity succinic acid.

US020190382328A1 discloses the conversion of wood based hemicellulose pre-hydrolysate into succinic acid using a heterogeneous acid catalyst in a biphasic system. The catalysts applied for this batch process are Amberlyst 15 and hydrogen peroxide. JP2006288361A refers to a batch process for producing succinic acid from agriculture and forestry based waste such as rice straw and waste wood. Suitable microorganisms are applied to accelerate the chemical reaction.

L. Yang et al., Carbohydrate Research, 346 (2011) 2304 - 2307, describe the conversion of cellulose to 1-(furan-2-yl)-2-hydroxyethanone in $ZnCl_2$ solution under microwave irradiation at 135 °C in a batch process. A. Lorente et al., International Journal of Biological Macromolecules, 237 (2023) 124149, refer to microwave radiation-assisted synthesis of levulinic acid from microcrystalline cellulose in a batch process.

CN104759244, CN101544758 and CN201227587 refer to a double-screw extruder reactor using microwave radiation to accelerate chemical reactions. Chlorination of polyvinyl chloride is described. A microwave generator is integrated into the twin-screw extruder via a waveguide in order to couple microwave radiation into a reaction zone. European patent application with the publication number EP4130532 and European patent application EP24154108.5 describe a pressure lock for an extruding apparatus allowing continuous operation of high pressure reactive extrusion processes. EP4130532 mentions reactive extrusion of biomass to obtain levulinic acid. However, EP4120532 is silent with respect to the application of microwaves. CN113149822B discloses a method for efficiently producing levulinic acid from cellulose resources which is characterized by comprising the following steps: step 1, preparing a strong acid catalyst, a cellulose raw material and acetic acid or an aqueous solution thereof into a reaction system; step 2, heating the reaction system prepared in the step 1 to 100-160 °C, and carrying out a reaction 10 min-30 h; step 3, after the reaction is finished, carrying out solid-liquid separation on the reaction system, wherein the solid obtained after separation is cellulose or other biomass components which are not completely reacted; a precipitant is added into the solution obtained after separation, and an acidic catalyst in the solution is neutralized and precipitated; solid-liquid separation is carried out again, and the obtained liquid is distilled to remove a solvent to obtain levulinic acid; the strong acid catalyst in the step 1 is sulfuric acid, phosphoric acid, methanesulfonic acid, trifluoromethanesulfonic acid, trichloroacetic acid or heteropolyacid; the heteropoly acid is phosphotungstic acid, phosphomolybdic acid, silicotungstic acid or silicomolybdic acid; the water content in the acetic acid aqueous solution in the step 1 is less than 20 wt %; and the precipitant in the step 3 comprises calcium hydroxide, calcium oxide, potassium hydroxide and ammonia water.

Thus, none of the prior art documents describes an efficient continuous production process of succinic and levulinic acid, in particular succinic acid, at high yield from biomass.

### Problem to be solved

The problem to be solved is the provision of a continuous production process of succinic and levulinic acid, in particular succinic acid, with short reaction times from biomass in high yield. Moreover, the production process should run on production apparatuses which can be set up directly at the producers of biomass. Large biomass producers are saw mills, where the wood waste, e.g. in the form of wood shavings or wood dust, is generated. In particular, the process should allow the usage of fresh undried wood, for example wood directly from wood logging operations.

## Summary of the invention

The present invention provides a method for producing succinic acid and levulinic acid, in particular succinic acid, from biomass, preferably biomass waste, by a continuous reactive process, which is carried out in a continuous flow reaction pipe, comprising at least one microwave generator, the method comprising the steps of:

- Continuously feeding an aqueous slurry of biomass into the housing interior of the continuous flow reaction pipe;
- Continuously feeding an acid catalyst into the housing interior of the continuous flow reaction pipe;
- Continuous reaction of the aqueous biomass slurry under electromagnetic microwave radiation at a temperature of more than 100 °C to achieve a pressure above normal pressure through the formation of water vapour;
- Continuous movement of the slurry towards the exit of the continuous flow reaction pipe;
- Continuous collection of the reaction products which exit the continuous flow reaction pipe;
- Separation of the obtained succinic acid from the reaction products,

wherein the acid catalyst comprises a Lewis acid in the form of metallic salt.
Further embodiments are described in the dependent claims.
It has been surprisingly found that the method as described herein can be used to produce in particular succinic acid at high yield, even in a continuous reaction process. This is surprising in the light of the prior art.

## Brief description of the figures

Preferred embodiments of the invention will be described in detail referring to the following figures:

Figure 1: Flow chart of the continuous production process of bio-based succinic and levulinic acid from biomass in a continuous flow reaction pipe.
Figure 2: Process design for continuous bio-based succinic and levulinic acid production process from biomass in a continuous flow reaction pipe with screws (extruder).

## Detailed description of the invention and exemplary embodiments

### 1. Continuous flow reaction pipe

The continuous process is carried out in a continuous flow reaction pipe, which is equipped with electromagnetic wave generators. Therefore, the investment costs are low. Any suitable design and shape can be principally used for the continuous flow reaction pipe. According to a preferred embodiment, the continuous flow reaction pipe has a round cross section. Due to their size, the mobile use of continuous flow reaction pipes can take place directly at biomass producers such as sawmills or forestry operations. Biomass can be processed into succinic acid, which is a raw material for the production of bio-based poly(butylene succinate), or into levulinic acid. In the continuous flow reaction pipe, a pressure above atmospheric pressure as well a continuous flow towards the pipe exit is maintained. A continuous flow can be achieved preferably by a screw, pump or gravity.

### 1.1 Continuous flow reaction pipe with screws

If screws are applied for achieving the continuous flow, a single screw extruder and preferably a twin-screw extruder are suitable as a continuous flow reaction pipe. Defined process parameters are used to break down the biomass into succinic and levulinic acid. Twin-screw extruders have been established in the plastics industry for decades. Due to their configuration options, they can be used very flexibly and can be purchased at any scale, from kg/h to t/h. They have several advantages over batch reactors when used for chemical reactions:

- High throughput with continuous operation.
- Easy scale-up due to standardisation of production models.
- Low investment costs for medium to large production plants.
- Mechanical decomposition of biomass using shear energy so that little mechanical pretreatment of biomass is required.

Continuous conveying and a wide range of configuration options of extruders offer good conditions for adapting the microwave batch process described below to a twin-screw extruder. The following twin-screw extruder configurations are suitable according to a preferred embodiment of the invention:

- The screw diameters are 25 mm to 180 mm.
- The twin screw extruder may be co-rotating as well as counter-rotating, preferably co-rotating.
- Every block of the twin screw extruder may have at least one gas inlet port, preferably two gas inlet ports.
- Gas inlet ports may be mounted with a microwave adapter made from heat resistant polymer, for example polyether ether ketone (PEEK), which serves as the barrier between microwave radiation and pressure and temperature from the twin-screw reactor.
- The steel of the twin-screw extruder has to be corrosion resistant to protect from catalyst induced steel corrosion.
- The L/D ratio of the twin-screw extruder is 40 to 100.

At least one microwave generator is coupled to the twin-screw extruder, preferably 2 to 20 microwave generators. The microwave generator may have a power of more than 200 W, for example more than 500 W and may have a frequency of 2.40 GHz to 2.50 GHz, preferably 2.45 GHz. Microwave generators are, for example, commercially available from Fricke und Mallah Microwave Technology GmbH. Further information with respect to the coupling of the microwave generators to the twin-screw extruder can be found in CN104759244, CN101544758, CN201227587.

The aqueous slurry of biomass is fed to the twin-screw extruder with the help of a commercially available hose pump. An example for a commercially available hose pump is Verderflex Dura Series, from Dura 15 to Dura 80, preferably model Dura 25 from Verder. Alternatively, the biomass may be fed directly into the interior housing of the extruder with a pressure lock. Examples for pressure locks are described in EP4130532 and in the European patent application EP24154108.5.

1.2 Continuous flow reaction pipe without screws

Instead of a screw, pumps and/or gravity can allow the continuous movement of the slurry towards the exit of the continuous flow reaction pipe. In the case of gravity, the feedstock may be in sufficient height to generate pressure in the pipe. A pipe without screws is cheaper than a conventional extruder. The at least one microwave generator is as described above for a continuous flow reaction pipe with screws.

2. Acids

According to the present invention, the acid catalyst comprises a Lewis acid in the form of metallic salt. According to an embodiment of the invention, the acid catalyst comprises a protonic acid, which is a chemical compound that forms protons in an aqueous solution, and the Lewis acid, which is a chemical compound that accepts electron pairs from another chemical compound.

The protonic acids may be selected from the group consisting of hydrochloric acid, sulfuric acid, phosphoric acid, nitric acid, sulfonic acid, sulfurous acid, hypochlorous acid, ammonium salts, boric acid, hydrobromic acid, perchloric acid, hydrofluoric acid, hydroiodic acid, nitrous acid, carbonous acid, chromous acid, selenic acid, molybdic acid, antimonic acid, silicic acid, tetraboric acid, carboxylic acids and mixtures thereof

The Lewis acids may be metallic salts, preferably selected from the group consisting of zinc salts, copper salts, chromium salts, lanthanum salts, tin salts, iron salts, cobalt salts, aluminium salts, scandium salts, titan salts, zirconium salts, iridium salts, strontium salts, vanadium salts, alkaline salts, earth alkaline salts and mixtures thereof, more preferably $Zn^{+2}$ salts and mixtures thereof, for example $ZnCl_2$

According to an embodiment, the concentration of the Lewis acid catalyst is at least 0.1 wt.% based on the weight of water of the aqueous chopped wood slurry. A suitable catalyst concentration of the slurry increases the yield of succinic or levulinic acid and reduces the reaction time.

According to the invention, the acid catalyst is or comprises at least one Lewis acid as described above, in particular at least one zinc salt. Most preferred the acid catalyst or the Lewis acid, respectively, is or comprises zinc chloride.

It has been surprisingly found that low acid catalyst concentrations, in particular Lewis acid concentrations in the continuous flow reaction pipe interior of less than about 8.0 wt.%, preferably less than about 5.5% wt.%, based on the weight of water of the aqueous slurry are advantageous to obtain succinic acid at high yield. According to a further preferred embodiment, the acid catalyst concentration (in particular zinc chloride) is at least 0.1 wt. % and less than about 8.0 wt.%, preferably less than about 5.5% wt.%, based on the weight of water of the aqueous slurry. Concentrations or at least 0.1 and at most 2 wt.%, or even from at least 0.1 to at most 1 wt.-%, based on the weight of water of the aqueous slurry, are even more preferred.

3. Biomass (wood waste)

According to the present invention, biomass is used in the continuous reactive process. According to a preferred embodiment, the biomass is a so-called lignocellulosic biomass. It is composed of two kinds of carbohydrate polymers, cellulose and hemicellulose, and an aromatic-rich polymer called lignin. Any biomass comprising cellulose, hemicelluloses, and lignin is commonly referred to as lignocellulosic biomass. Being a composite of three very different components makes the processing of lignocellulose challenging. However, it was surprisingly found that production of succinic acid at high yields is possible directly from an aqueous slurry of biomass (lignocellulosic biomass) in one continuous reactive process disclosed herein. Lignocellulosic biomass can be broadly classified as virgin biomass, waste biomass, and energy crops. Waste biomass (or biomass waste) is produced as a low value byproduct of various industrial sectors such as agriculture (corn stover, sugarcane bagasse, straw etc.) and forestry (saw mill and paper mill discards). Within the present invention, waste biomass is preferred as biomass (lignocellulosic biomass), in particular wood waste biomass.

According to a preferred embodiment of the invention, chopped wood waste (as waste biomass) is present or used in the form of pellets or particles. As the wood waste particles or pellets do not need any prior treatment before the reactive extrusion, the process is very efficient and saves costs.

The chopped wood waste can be directly fed into the continuous flow reaction pipe for example with the help of a pressure lock, or in the form of an aqueous slurry with the help of a hose pump. According to an embodiment, the loading of the wood waste in the aqueous slurry is at least 0.3 wt.% based on the weight of water of the slurry.

According to a preferred embodiment of the invention, the biomass loading in the continuous flow reaction pipe interior is at least 0.3 wt.% and less than about 18 wt.%, more preferably less than about 5 wt.-%, even more preferably at most about 3.3 wt.-%, based on the weight of water of the aqueous slurry.

According to a specific preferred embodiment, about 1.0 wt.% or less of zinc chloride, based on the weight of water of the aqueous slurry, are used as acid catalyst, and the biomass loading in the continuous flow reaction pipe interior is about 3.3 wt.% or less, in particular about 2.0 wt.% or less, based on the weight of water of the aqueous slurry. Further preferred, at least 0.1 wt.% and at most about 1.0 wt.% of zinc chloride, based on the weight of water of the aqueous slurry, are used as acid catalyst, and the biomass loading in the continuous flow reaction pipe interior is at least 0.3 wt.% and at most 3.3 wt.%, in particular is at least 0.3 wt.% and at most about 2.0 wt.%, based on the weight of water of the aqueous slurry. It was surprisingly found that this allows a particularly high yield of succinic acid.

The following embodiments are also disclosed.

1. A method for producing succinic acid and levulinic acid, in particular succinic acid, from biomass by a continuous reactive process in a continuous flow reaction pipe comprising at least one microwave generator, the method comprising the steps of:

- Continuously feeding an aqueous slurry comprising biomass into the interior housing of the continuous flow reaction pipe;
- Continuously feeding an acid catalyst in the interior housing of the continuous flow reaction pipe;
- Continuous reaction of the slurry under electromagnetic microwave radiation at a temperature above 100 °C resulting in an elevated pressure through the formation of water vapor;
- Continuous movement of the slurry towards the exit of the continuous flow reaction pipe;
- Continuous collection of the reaction products which exit the continuous flow reaction pipe;
- Separation of the obtained succinic acid and levulinic acid from the reaction products.

Thus, one further disclosure broadly encompasses a method of producing succinic and levulinic acid from biomass.

2. The method according to embodiment 1, wherein the movement of the slurry towards the exit of the continuous flow reaction pipe is achieved by a screw, a pump and/or gravity.

3. The method according to any preceding embodiment, wherein the biomass is biomass waste.

4. The method according to any preceding embodiment, wherein the biomass is wood, plants, cellulose, hemicellulose or lignin.

5. The method according to any preceding embodiment, characterised in that the biomass is chopped wood, preferably wood waste in the form of pellets or particles, more preferably wood waste in the form untreated sawdust or wood shavings.

6. The method according to any preceding embodiment,
characterised in that the acid catalyst comprises a protonic acid, which is a chemical compound that forms protons in an aqueous solution, and/or a Lewis acid, which is a chemical compound that accepts electron pairs from another chemical compound.

7. The method according to embodiment 6, wherein the Lewis acids are metallic salts, preferably selected from the group consisting of zinc salts, copper salts, chromium salts, lanthanum salts, tin salts, iron salts, cobalt salts, aluminium salts, scandium salts, titan salts, zirconium salts, iridium salts, strontium salts, vanadium salts, alkaline salts, earth alkaline salts and mixtures thereof, more preferably zinc salts and mixtures thereof.

8. The method according to embodiments 6 and 7, wherein the protonic acid is selected from the group consisting of hydrochloric acid, sulfuric acid, phosphoric acid, nitric acid, sulfonic acid, sulfurous acid, hypochlorous acid, ammonium salts, boric acid hydrobromic acid, perchloric acid, hydrofluoric acid, hydroiodic acid, nitrous acid, carbonous acid, chromous acid, selenic acid, molybdic acid, antimonic acid, silicic acid, tetraboric acid, carboxylic acids and mixtures thereof

9. The method according to embodiment 2, characterised in that the screw is the screw of an extruder with more than 1, preferably 2 to 20 microwave generators, having a power of at least 200 W, preferably of at least 500 W.

10. The method according to embodiment 9, characterised in that the screw speed is less than 400 rpm, and/or in that the ratio L/D is 40 to 100.

11. The method according to embodiments 9 and 10, characterised in that the extruder is a twin screw extruder.

12. The method according to any preceding embodiment, characterised in that the aqueous slurry of the biomass is added by a hose pump.

13. The method according to any preceding embodiment, characterised in that the biomass loading in the continuous flow reaction pipe interior is at least 0.3 wt.% based on the weight of water of the aqueous slurry.

14. The method according to any preceding embodiment, characterised in that the temperature is at least 150 °C;

15. The method according to any preceding embodiment, characterised in that the Lewis acid catalyst concentration in the continuous flow reaction pipe interior is at least 0.1 wt.% based on the weight of water of the aqueous slurry.

16. The method according to any preceding embodiment, characterised in that the succinic acid and levulinic acid are separated by a rectification column and/or a spray drying system.

17. The method according to any preceding embodiment, characterized in that the cooling water used to cool the liquid product flow comprising the succinic acid and levulinic acid downstream of the continuous flow reaction pipe is used for heating the continuous flow reaction pipe to the desired temperature.

18. The method according to any preceding embodiment,

characterised in that the succinic acid is further transformed into butane diol.

According to a further disclosure it was surprisingly found that the use of zinc chloride as acid catalyst at a low concentration of less than 2 wt.% as defined above, under electromagnetic microwave radiation at a temperature above 150 °C resulting in an elevated pressure through the formation of water vapour, together with a low (lignocellulosic) biomass concentration of at least 0.3 wt.% to at most 3.3 wt.%, based on the weight of water of the aqueous slurry, works particularly well not only in a continuous reactive process as described, but also in a batch-type reaction (in a closed reactor).

A further alternative disclosure is thus directed to a method for producing succinic acid from lignocellulosic biomass, in particular waste wood biomass, by a batch-type reactive process in a reactor comprising at least one microwave generator, the method comprising the steps of:

- feeding an aqueous slurry comprising biomass into the interior housing of the reactor;

- feeding an acid catalyst in the interior housing of the reactor;
- reaction of the slurry under electromagnetic microwave radiation at a temperature above 100 °C, in particular 150 °C resulting in an elevated pressure in the reactor through the formation of water vapor;
- separation of the obtained succinic acid.

Brief description of the figures

The invention will be described in detail referring to the following figures.

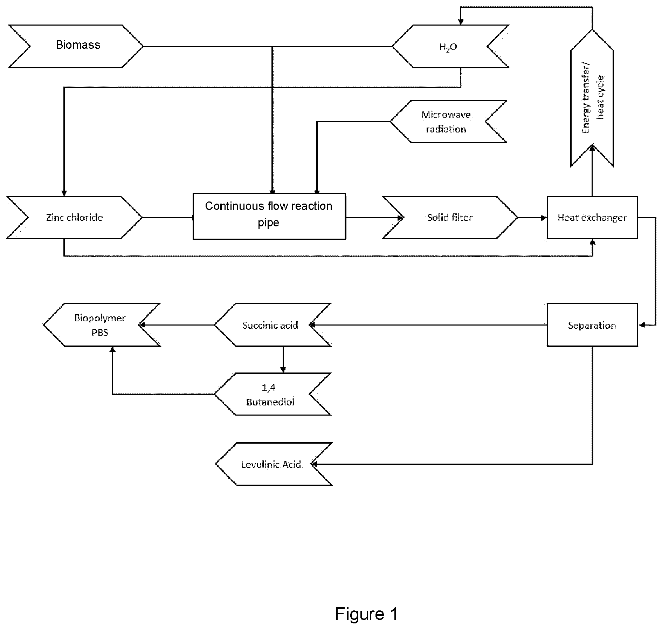

Figure 1: Flow chart of the continuous production process of bio-based succinic and levulinic acid from wood waste in a continuous flow reaction pipe

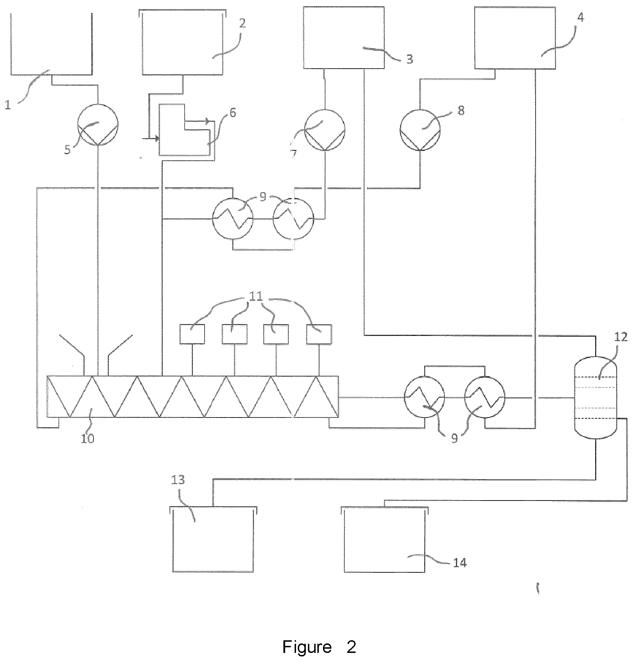

Figure 2: Process design for continuous bio-based succinic and levulinic acid production process from wood waste in a continuous flow reaction pipe with screws (extruder)

4. Batch and twin extruder experiments with spruce wood

4.1 Raw Materials

Spruce wood contains on average 29 wt.% lignin, 26 wt.% hemicellulose, and 43 wt.% cellulose. The remaining 2 wt.% are made up of extractives and ash. Spruce is one of the most common wood species in Europe and is classified as a softwood. The spruce wood shavings used in the experiments were accumulated from wood residues or saw dust. These were used without any further pretreatment steps. Any additional drying or grinding steps were omitted.
Zinc chloride (anhydrate, $\geq$ 95 %, VWR), was used in the microwave experiments. Levulinic acid (for synthesis, Sigma Aldrich), formic acid (99 %, VWR), 2-oxogliutaric acid (for biochemistry, Sigma Aldrich) and succinic acid (99.6 %, VWR) were used as external standards for the analysis. The supra pure hydrochloric acid (30 %, Merck) as well as the nitric acid (69 %, Roth) were used in cleaning runs for the microwave pressure vessels.

4.2 Production of succinic and levulinic acid in a batch process (not according to the invention) from wood waste

The hydrolysis of wood was carried out using a microwave system. For this purpose, a Multiwave 5000 from Anton Paar was employed, along with the corresponding rotor (20SVT50) and Teflon pressure vessels (SVT50). For each sample, 0.20 g of spruce wood was weighed into the Teflon vessels. 6.0 ml of an aqueous 1.0 wt.% $ZnCl_2$ solution having a concentration of $ZnCl_2$ of 1.0 g/100 g of water was added. Combining 0.20 g of spruce wood with 6.0 ml of this catalyst solution resulted in a spruce loading of 0.20 g per 6 g of water, corresponding to a biomass loading of 3.3 wt.%. The target temperature of the microwave was set to 200 °C. The pressure reached for this temperature was 1.5 MPa (15 bar). The heating-up time for all setups was 10 minutes. For each sample set, the reaction time at 200 °C was varied (2 minutes, 5 minutes, 10 minutes). After the reaction, the samples were cooled to 65 °C. Each experiment was performed in quadruplicate. After the completion of the microwave program, the treated samples were transferred to plastic containers for storage. The vessels were rinsed multiple times with distilled water. After each experimental run, a cleaning procedure was performed. For this purpose, the vessels were cleaned at 180°C in the microwave for 20 minutes using 4 ml of distilled water, 2 ml of hydrochloric acid, and 3 ml of nitric acid.

4.3 Analytical Methods

For the quantitative determination of the hydrolysis products, a high performance liquid chromatography (HPLC) analysis was conducted. Calibration was performed utilizing external standards. Retention time matching was used for qualitative determination. The qualitative determination was carried out with an liquid chromatography-mass spectrometry (LC/MS) analysis. Using the mass spectra, the respective substances in the samples were identified. All samples from the microwave treatment were diluted for analysis in a 1:20 ratio for both the HPLC and LC/MS systems. The parameters applied for both the HPLC-, and the LC/MS-analysis are listed in Table 1.

Table 1: Parameters for the analytical methods HPLC and LC/MS

| Parameters | HPLC | LC/MS |
|---|---|---|
| System | PerkinElmer Series 200 | Agilent LC/MSD iQ Series |
| Column | Aminex® HPX-87H Column, Bio-Rad | |
| Column Parameters | 300 x 7,8 mm; 35°C; Particle size: 9 $\mu$ m | |

(continued)

| Parameters | HPLC | LC/MS |
|---|---|---|
| Method | Isocratic, Negative Ion Mode for LC/MS | |
| Time | 25 min | 15 min |
| Injection Volume | 20 $\mu$l | |
| Mobile Phase | 0.005 N $H_2SO_4$ | 0.5 % v/v Formic Acid |
| Flow | 0.6 ml/min | |
| Scan Area | - | 50 - 450 m/z |
| Gas Temperature | - | 285°C |
| Gas Flow | - | 13 l/min |
| Fragmentor | - | 75 V |
| Capillary | - | 3 kV |

4.4 Batch examples 1 to 3 (not according to the invention)

All batch examples were carried out using spruce wood without further purification as raw material, which was added to aqueous $ZnCl_2$ solutions to obtain aqueous spruce slurries having spruce loadings of 3.3 wt.% and 18 wt.% based on the weight of water in the aqueous slurry. The aqueous $ZnCl_2$ solutions had concentrations of 1.0 wt.%, 5.5 wt.% and 8.0 wt.% based on the weight of water. The reaction times were 2.0, 5.0 and 10 minutes. During subsequent LC/MS and HPLC analyses, a total of 11 different m/z (mass-to-charge ratio) values and 8 different peaks were identified. Among them, the four substances, namely levulinic acid, succinic acid, formic acid, and 2-oxoglutaric acid, were clearly identified and quantitatively analysed. The results of the chemical concentrations in the analysis sample were subsequently adjusted based on the dilution factor (20), the amount of water in the sample, and the weighed amount of wood. The results provide the yield per gram of spruce wood. The following example calculation according to Formula 1 illustrates the calculation of the succinic acid yield. The first factor in the spruce loading is determined by the applied calibration curve for succinic acid.

## Formula 1:

$$Y_{SA} = \frac{\frac{(y-703.89)}{981915} * 20 * V_{Sample}}{1000 * m_{Wood}}$$

$Y_{SA}$   Yield of succinic acid in g/g$_{Wood}$
$y$   Peak area in $\mu$ Vs
$V_{Sample}$   Sample volume in ml
$m_{Wood}$   Amount of spruce wood in g

Table 2: Overview of the loading of wood spruce, amount of aqueous $ZnCl_2$ solution, concentration of aqueous $ZnCl_2$ solution, temperature, reaction time, yields of succinic acid calculated according to Formula 1

| Sample | wood spruce [wt.%] | Aqueous $ZnCl_2$ solution [ml] | Conc. $ZnCl_2$ [wt.%] | Temp. [°C] | Reaction time [min] | Yield [wt.%] |
|---|---|---|---|---|---|---|
| Ex1 | 3.3 | 6.0 ml | 1.0 | 200 °C | 2.0 | 59.8 |
| Ex2 | 3.3 | 6.0 ml | 1.0 | 200 °C | 5.0 | 43.7 |
| Ex3 | 3.3 | 6.0 ml | 1.0 | 200 °C | 10 | 19.8 |

Batch examples 1 to 3 of Table 2 show, that the fine tuning of the reaction time from 2.0 to 10 minutes is important to achieve high yields of succinic acid in the batch process. A reaction temperature of 200 °C corresponds to a pressure of 1.5 MPa or 15 bar.
The examples performed also showed that too high loadings of biomass resulted in a lower yield of succinic acid. Thus, by changing the biomass concentration in Ex1 above (from 3.3 wt.%) to 5 or 10 wt.%, respectively, the yield of succinic acid dropped from 59.8 wt.% to about 31.5 wt.% and about 10.2 wt. %, respectively.

A low conversion of wood spruce in succinic acid was also observed in case the reaction temperature was too low to achieve sufficient pressure (due to a low temperature below 100°C) and apparently thus sufficient activity of the catalyst $ZnCl_2$.

As shown in the Table above, too long times caused also a lower yield of succinic acid in the process.

Similarly, too high catalyst concentrations caused also a lower yield of succinic acid in the process. Thus, by changing the catalyst concentration in Ex1 above (from 1.0 wt.%) to 5% wt.%, the yield of succinic acid dropped significantly.

4.5 Upscaling to a twin screw extruder

The flow chart of the production process in a continuous flow reaction pipe of bio based succinic and levulinic acid by reactive processing of wood waste with $ZnCl_2$ as exemplary catalyst is shown in Figure 1. Figure 2 shows the embodiment of a continuous flow reaction pipe with screws .

As a continuous flow reaction pipe with screws, a twin screw extruder (10) is applied, the aqueous slurry of chopped wood, for example wood shavings or wood saw (1), is converted into succinic and levulinic using microwave generators (11), an elevated temperature and the Lewis acid $ZnCl_2$.

The temperature of the twin screw reactor amounts to more than 100 °C, preferably least 150 °C. The higher the temperature, the higher the pressure within the twin-screw reactor due to water vapor.

The screw speed is less than 400 rpm, preferably less than 200 rpm.

Chopped wood waste and water are mixed to form a slurry and fed into the extruder by the hose pump (5). The loading of wood waste based on the weight of water amounts to least 0.3 wt.%.

A Lewis acid catalyst, for example $ZnCl_2$, is added using a dosing pump (6). The catalyst concentration in the interior housing in the extruder amounts, for example, to at least 0.1 wt.%. 0.1 wt.% catalyst concentration corresponds to 0.1 g of catalyst in 100 ml of water of the chopped wood slurry. Alternatively, the catalyst and the waste wood slurry may be added together.

The high conversion rates achieved with processing times below 30 minutes are partly due to the strong influence of microwave radiation on the cellulose structure. Electromagnetic radiation in the microwave frequency range causes the breakdown of hydrogen bonds in the cellulose molecule, which promotes the acidic hydrolysis of the biopolymer and thus accelerates the production of succinic and levulinic acid. Microwave radiation is crucial in enabling short reaction times and high yields.

To improve the overall efficiency of the process, heat exchangers (9) and an associated circulation pump (7) can be integrated into the process. This allows to extract heat energy from the liquid flow downstream of the twin screw extruder and cool it down using secondary water. The secondary water can be used to heat the twin screw reactor via the cooling holes. This heats the reaction water and requires less electrical energy, which improves the overall efficiency of the process.

To filter succinic and levulinic acid out of the product stream, for example, a rectification column/distillation column (12) with a subsequent spray drying unit may be applied (13). Succinic acid crystallises when water is quickly removed, which allows good separation with spray drying. Other chemical products contained and dissolved in the product stream include the catalyst and the product levulinic acid (14). Water (3) is fed back into the system via the distillation column and a positive displacement pump (7), while other platform chemicals such as levulinic acid are separated.

4.6 Twin screw extruder examples with spruce wood

Twin screw extruder example 1

- Residence time: 3 min,
- Temperature: 200°C,
- Biomass loading: 2 weight %, the remainder is water and catalyst
- Biomass: spruce wood chips, average particle size 2.5 mm
- Catalyst: $ZnCl_2$,
- Catalyst concentration: 0.5% by weight,
- Microwave power: 3200 W
- Yield succinic acid: 35.6 %

Twin screw extruder example 2

- Residence time: 5 min,
- Temperature: 200°C,
- Biomass loading: 2 weight %, the remainder is water and catalyst
- Biomass: spruce wood chips, average particle size 2.5 mm
- Catalyst: $ZnCl_2$,
- Catalyst concentration: 0.5% by weight,
- Microwave power: 3200 W
- Yield succinic acid acid: 22.1 %

Again, a too high biomass concentration or acid catalyst concentration lead to a lower succinic acid yield.

## Claims

1. A method for producing succinic acid and levulinic acid, in particular succinic acid, from biomass by a continuous reactive process in a continuous flow reaction pipe comprising at least one microwave generator, the method comprising the steps of:

   - Continuously feeding an aqueous slurry comprising biomass into the interior housing of the continuous flow reaction pipe;
   - Continuously feeding an acid catalyst in the interior housing of the continuous flow reaction pipe;
   - Continuous reaction of the slurry under electromagnetic microwave radiation at a temperature above 100 °C resulting in an elevated pressure through the formation of water vapor;
   - Continuous movement of the slurry towards the exit of the continuous flow reaction pipe;
   - Continuous collection of the reaction products which exit the continuous flow reaction pipe;
   - Separation of the obtained succinic acid and levulinic acid, in particular the succinic acid, from the reaction products,

   wherein the acid catalyst comprises a Lewis acid, and wherein the Lewis acid is a metallic salt.

2. The method according to claim 1, wherein the acid catalyst comprises a zinc salt, in particular zinc chloride.

3. The method according to claim 1 or 2, wherein the acid catalyst concentration, in particular a Lewis acid concentration in the continuous flow reaction pipe interior is at least 0.1 wt.% and less than about 8.0 wt. %, preferably less than about 5.5% wt.%, based on the weight of water of the aqueous slurry.

4. The method according to any of the preceding claims, wherein the biomass loading in the continuous flow reaction pipe interior is at least 0.3 wt.% and less than about 18 wt.%, based on the weight of water of the aqueous slurry.

5. The method according to any of the preceding claims, wherein at least 0.1 wt.% and at most about 1.0 wt.% of zinc chloride, based on the weight of water of the aqueous slurry, are used as acid catalyst, and the biomass loading in the continuous flow reaction pipe interior is at least 0.3 wt.% and at most 3.3 wt.%, in particular is at least 0.3 wt.% and at most about 2.0 wt.%, based on the weight of water of the aqueous slurry.

6. The method according to claim 1, wherein the movement of the slurry towards the exit of the continuous flow reaction pipe is achieved by a screw, a pump and/or gravity.

7. The method according to any preceding claim, wherein the biomass is biomass waste,

   and/or wherein the biomass is wood, plants, cellulose, hemicellulose or lignin,
   and/or wherein the biomass is chopped wood, preferably wood waste in the form of pellets or particles, more preferably wood waste in the form untreated sawdust or wood shavings.

8. The method according to any preceding claim, **characterised in that** the acid catalyst comprises a protonic acid and a Lewis acid.

9. The method according to any preceding claim, wherein the Lewis acids are metallic salts selected from the group consisting of zinc salts, copper salts, chromium salts, lanthanum salts, tin salts, iron salts, cobalt salts, aluminium salts, scandium salts, titan salts, zirconium salts, iridium salts, strontium salts, vanadium salts, alkaline salts, earth alkaline salts and mixtures thereof, more preferably zinc salts and mixtures thereof.

10. The method according to claim 8 or 9, wherein the protonic acid is selected from the group consisting of hydrochloric acid, sulfuric acid, phosphoric acid, nitric acid, sulfonic acid, sulfurous acid, hypochlorous acid, ammonium salts, boric acid hydrobromic acid, perchloric acid, hydrofluoric acid, hydroiodic acid, nitrous acid, carbonous acid, chromous acid, selenic acid, molybdic acid, antimonic acid, silicic acid, tetraboric acid, carboxylic acids and mixtures thereof

11. The method according to claim 6,

   **characterised in that** the screw is the screw of an extruder with more than 1, preferably 2 to 20 microwave generators, having a power of at least 200 W, preferably of at least 500 W, optionally wherein the screw speed is less than 400 rpm, and/or **in that** the ratio L/D is 40 to 100,
   and/or wherein the extruder is a twin screw extruder.

12. The method according to any preceding claim, **characterised in that** the aqueous slurry of the biomass is added by a hose pump,

   and/or wherein the biomass loading in the continuous flow reaction pipe interior is at least 0.3 wt.% based on the weight of water of the aqueous slurry,
   and/or wherein the temperature is at least 150 °C,
   and/or wherein the reaction time is less than 10 min, preferably less than 5 min, more preferably at most 3 min.

13. The method according to any preceding claim, **characterised in that** the Lewis acid catalyst concentration in the continuous flow reaction pipe interior is at least 0.1 wt.% based on the weight of water of the aqueous slurry.

14. The method according to any preceding claim, **characterised in that** the succinic acid and levulinic acid are separated by a rectification column and/or a spray drying system,
and/or wherein the cooling water used to cool the liquid product flow comprising the succinic acid downstream of the continuous flow reaction pipe is used for heating the continuous flow reaction pipe to the desired temperature.

15. The method according to any preceding claim, **characterised in that** the succinic acid is further transformed into butane diol.


**Patentansprüche**

1. Verfahren zur Herstellung von Bernsteinsäure und Lävulinsäure, insbesondere Bernsteinsäure, aus Biomasse durch einen kontinuierlichen Reaktionsprozess in einem Durchflussreaktionsrohr, das mindestens einen Mikrowellengenerator umfasst, wobei das
Verfahren die folgenden Schritte umfasst:

   - kontinuierliches Einleiten einer wässrigen Aufschlämmung, die Biomasse enthält, in das Innengehäuse des Durchflussreaktionsrohrs;
   - kontinuierliches Einbringen eines Säurekatalysators in das Innengehäuse des Durchflussreaktionsrohrs;
   - kontinuierliche Reaktion der Aufschlämmung unter elektromagnetischer Mikrowellenstrahlung bei einer Temperatur über 100 °C, was durch die Bildung von Wasserdampf zu einem erhöhten Druck führt;
   - kontinuierliche Bewegung der Aufschlämmung in Richtung des Auslasses des Durchflussreaktionsrohrs;
   - kontinuierliches Auffangen der Reaktionsprodukte, die das Durchflussreaktionsrohr verlassen;
   - Abtrennung der erhaltenen Bernsteinsäure und Levulinsäure, insbesondere der Bernsteinsäure, aus den Reaktionsprodukten,

   wobei der Säurekatalysator eine Lewis-Säure umfasst und wobei die Lewis-Säure ein Metallsalz ist.

**2.** Verfahren nach Anspruch 1, wobei der Säurekatalysator ein Zinksalz, insbesondere Zinkchlorid, umfasst.

**3.** Verfahren nach Anspruch 1 oder 2, wobei die Säurekatalysatorkonzentration, insbesondere die Lewis-Säure-Konzentration, im Inneren des Durchflussreaktionsrohrs mindestens 0,1 Gew.-% und weniger als etwa 8,0 Gew.-%, vorzugsweise weniger als etwa 5,5 Gew.-%, beträgt, bezogen auf das Gewicht an Wasser der wässrigen Aufschlämmung.

**4.** Verfahren nach einem der vorstehenden Ansprüche, wobei die Biomassebeladung im Inneren des Durchflussreaktionsrohrs mindestens 0,3 Gew.-% und weniger als etwa 18 Gew.-%, bezogen auf das Gewicht an Wasser der wässrigen Aufschlämmung, beträgt.

**5.** Verfahren nach einem der vorstehenden Ansprüche, wobei mindestens 0,1 Gew.-% und höchstens etwa 1,0 Gew.-% Zinkchlorid, bezogen auf das Gewicht an Wasser der wässrigen Aufschlämmung, als Säurekatalysator verwendet werden und die Biomassebeladung im Inneren des Durchflussreaktionsrohrs mindestens 0,3 Gew.-% und höchstens 3,3 Gew.-% beträgt, insbesondere mindestens 0,3 Gew.-% und höchstens etwa 2,0 Gew.-%, bezogen auf das Gewicht an Wasser der wässrigen Aufschlämmung, beträgt.

**6.** Verfahren nach Anspruch 1, wobei die Bewegung der Aufschlämmung in Richtung des Auslasses des Durchflussreaktionsrohrs durch eine Schnecke, eine Pumpe und/oder Schwerkraft bewirkt wird.

**7.** Verfahren nach einem der vorstehenden Ansprüche, wobei die Biomasse Biomasseabfälle sind,

und/oder wobei die Biomasse Holz, Pflanzen, Zellulose, Hemicellulose oder Lignin ist,
und/oder wobei die Biomasse gehacktes Holz ist, vorzugsweise Holzabfälle in Form von Pellets oder Partikeln, noch bevorzugter Holzabfälle in Form von unbehandeltem Sägemehl oder Holzspänen.

**8.** Verfahren nach einem der vorstehenden Ansprüche, **dadurch gekennzeichnet, dass** der Säurekatalysator eine protonische Säure und eine Lewis-Säure umfasst.

**9.** Verfahren nach einem der vorstehenden Ansprüche, wobei die Lewis-Säuren Metallsalze sind, ausgewählt aus der Gruppe bestehend aus Zinksalzen, Kupfersalzen, Chromsalzen, Lanthansalzen, Zinnsalzen, Eisensalzen, Kobaltsalzen, Aluminiumsalzen, Scandiumsalzen, Titansalzen, Zirkoniumsalzen, Iridiumsalzen, Strontiumsalzen, Vanadiumsalzen, Alkalisalzen, Erdalkalisalzen und Mischungen davon, vorzugsweise Zinksalzen und Mischungen davon, ausgewählt sind.

**10.** Verfahren nach Anspruch 8 oder 9, wobei die protonische Säure aus der Gruppe ausgewählt ist, die aus Salzsäure, Schwefelsäure, Phosphorsäure, Salpetersäure, Sulfonsäure, schwefliger Säure, hypochloriger Säure, Ammoniumsalzen, Borsäure, Bromwasserstoffsäure, Perchlorsäure, Flusssäure, Iodwasserstoffsäure, salpetrige Säure, Karbonsäure, Chromsäure, Selensäure, Molybdänsäure, Antimonsäure, Kieselsäure, Tetraborsäure, Carbonsäuren und Mischungen davon

**11.** Verfahren nach Anspruch 6, **dadurch gekennzeichnet, dass** die Schnecke die Schnecke eines Extruders mit mehr als 1, vorzugsweise 2 bis 20 Mikrowellengeneratoren ist, die eine Leistung von mindestens 200 W, vorzugsweise von mindestens 500 W, aufweisen, wobei gegebenenfalls die Schneckendrehzahl weniger als 400 U/min beträgt, und/oder das Verhältnis L/D 40 bis 100 beträgt, und/oder wobei der Extruder ein Doppelschneckenextruder ist.

**12.** Verfahren nach einem der vorstehenden Ansprüche, **dadurch gekennzeichnet, dass** die wässrige Aufschlämmung der Biomasse mittels einer Schlauchpumpe zugeführt wird,

und/oder wobei der Biomasseanteil im Inneren des Durchflussreaktionsrohrs mindestens 0,3 Gew.-%, bezogen auf das Gewicht an Wasser der wässrigen Aufschlämmung, beträgt,
und/oder wobei die Temperatur mindestens 150 °C beträgt,
und/oder wobei die Reaktionszeit weniger als 10 min, vorzugsweise weniger als 5 min, weiter bevorzugt höchstens 3 min beträgt.

**13.** Verfahren nach einem der vorstehenden Ansprüche, **dadurch gekennzeichnet, dass** die Lewis-Säure-Katalysatorkonzentration im Inneren des Durchflussreaktionsrohrs mindestens 0,1 Gew.-%, bezogen auf das Gewicht an Wasser der wässrigen Aufschlämmung, beträgt.

**14.** Verfahren nach einem der vorstehenden Ansprüche, **dadurch gekennzeichnet, dass** die Bernsteinsäure und die Lävulinsäure mittels einer Rektifikationskolonne und/oder einer Sprühtrocknungsanlage getrennt werden, und/oder wobei das Kühlwasser, das zur Kühlung des die Bernsteinsäure enthaltenden flüssigen Produktstroms stromabwärts des Durchflussreaktionsrohrs verwendet wird, zum Erwärmen des Durchflussreaktionsrohrs auf die gewünschte Temperatur genutzt wird.

**15.** Verfahren nach einem der vorstehenden Ansprüche, **dadurch gekennzeichnet, dass** die Bernsteinsäure weiter in Butandiol umgewandelt wird.

**Revendications**

**1.** Procédé de production d'acide succinique et d'acide lévulinique, en particulier d'acide succinique, à partir de biomasse par un procédé réactif continu dans un tube de réaction à écoulement continu comprenant au moins un générateur de micro-ondes, le procédé comprenant les étapes :

- d'introduction continue d'une suspension aqueuse comprenant de la biomasse dans le boîtier intérieur du tube de réaction à écoulement continu ;
- d'introduction continue d'un catalyseur acide dans le boîtier intérieur du tube de réaction à écoulement continu ;
- de réaction continue de la suspension sous rayonnement micro-onde électromagnétique à une température supérieure à 100°C, entraînant une pression élevée par la formation de vapeur d'eau ;
- de mouvement continu de la suspension vers la sortie du tube de réaction à écoulement continu ;
- de collecte continue des produits de réaction qui sortent du tube de réaction à écoulement continu ;
- de séparation de l'acide succinique et de l'acide lévulinique obtenus, en particulier de l'acide succinique, des produits de réaction,

dans lequel le catalyseur acide comprend un acide de Lewis, et dans lequel l'acide de Lewis est un sel métallique.

**2.** Procédé selon la revendication 1, dans lequel le catalyseur acide comprend un sel de zinc, en particulier du chlorure de zinc.

**3.** Procédé selon la revendication 1 ou 2, dans lequel la concentration de catalyseur acide, en particulier la concentration d'acide de Lewis, dans la partie intérieure de tube de réaction à écoulement continu est d'au moins 0,1% en poids et inférieure à environ 8,0% en poids, de préférence inférieure à environ 5,5% en poids, par rapport au poids d'eau de la suspension aqueuse.

**4.** Procédé selon l'une des revendications précédentes, dans lequel la charge de biomasse dans la partie intérieure de tube de réaction à écoulement continu est d'au moins 0,3% en poids et inférieure à environ 18% en poids, par rapport au poids d'eau de la suspension aqueuse.

**5.** Procédé selon l'une des revendications précédentes, dans lequel au moins 0,1% en poids et au plus environ 1,0% en poids de chlorure de zinc, par rapport au poids d'eau de la suspension aqueuse, sont utilisés comme catalyseur acide, et la charge de biomasse dans la partie intérieure de tube de réaction à écoulement continu est d'au moins 0,3% en poids et d'au plus 3,3% en poids, en particulier est d'au moins 0,3% en poids et d'au plus environ 2,0% en poids, par rapport au poids d'eau de la suspension aqueuse.

**6.** Procédé selon la revendication 1, dans lequel le mouvement de la suspension vers la sortie du tube de réaction à écoulement continu est réalisé par une vis, une pompe et/ou la gravité.

**7.** Procédé selon l'une des revendications précédentes, dans lequel la biomasse représente des déchets de biomasse,

et/ou dans lequel la biomasse est du bois, des plantes, de la cellulose, de l'hémicellulose ou de la lignine, et/ou dans lequel la biomasse est du bois broyé, de préférence des déchets de bois sous forme de granulés ou de particules, plus préférablement des déchets de bois sous forme de sciure ou de copeaux de bois non traités.

**8.** Procédé selon l'une des revendications précédentes, **caractérisé en ce que** le catalyseur acide comprend un acide protonique et un acide de Lewis.

9. Procédé selon l'une des revendications précédentes, dans lequel les acides de Lewis sont des sels métalliques choisis dans le groupe constitué par les sels de zinc, les sels de cuivre, les sels de chrome, les sels de lanthane, les sels d'étain, les sels de fer, les sels de cobalt, les sels d'aluminium, les sels de scandium, les sels de titane, les sels de zirconium, les sels d'iridium, les sels de strontium, les sels de vanadium, les sels alcalins, les sels alcalino-terreux et des mélanges de ceux-ci, plus préférablement les sels de zinc et des mélanges de ceux-ci.

10. Procédé selon la revendication 8 ou 9, dans lequel l'acide protonique est choisi dans le groupe constitué par l'acide chlorhydrique, l'acide sulfurique, l'acide phosphorique, l'acide nitrique, l'acide sulfonique, l'acide sulfureux, l'acide hypochloreux, les sels d'ammonium, l'acide borique, l'acide bromhydrique, l'acide perchlorique, l'acide fluorhydrique, l'acide iodhydrique, l'acide nitreux, l'acide carboneux, l'acide chromique, l'acide sélénique, l'acide molybdique, l'acide antimonique, l'acide silicique, l'acide tétraborique, les acides carboxyliques et des mélanges de ceux-ci.

11. Procédé selon la revendication 6,

   caractérisé en ce que la vis est la vis d'une extrudeuse avec plus de 1, de préférence 2 à 20 générateurs de micro-ondes, ayant une puissance d'au moins 200 W, de préférence d'au moins 500 W, éventuellement dans lequel la vitesse de la vis est inférieure à 400 tr/min, et/ou en ce que le rapport L/D est compris entre 40 et 100, et/ou dans lequel l'extrudeuse est une extrudeuse à double vis.

12. Procédé selon l'une des revendications précédentes, caractérisé en ce que la suspension aqueuse de la biomasse est ajoutée par une pompe à tuyau,

   et/ou dans lequel la charge de biomasse dans la partie intérieure de tube de réaction à écoulement continu est d'au moins 0,3% en poids par rapport au poids d'eau de la suspension aqueuse,
   et/ou dans lequel la température est d'au moins 150°C,
   et/ou dans lequel le temps de réaction est inférieur à 10 min, de préférence inférieur à 5 min, plus préférablement d'au plus 3 min.

13. Procédé selon l'une des revendications précédentes, caractérisé en ce que la concentration de catalyseur acide de Lewis dans la partie intérieure de tube de réaction à écoulement continu est d'au moins 0,1% en poids par rapport au poids d'eau de la suspension aqueuse.

14. Procédé selon l'une des revendications précédentes, caractérisé en ce que l'acide succinique et l'acide lévulinique sont séparés par une colonne de rectification et/ou un système de séchage par atomisation, et/ou dans lequel l'eau de refroidissement utilisée pour refroidir le flux de produit liquide comprenant l'acide succinique en aval du tube de réaction à écoulement continu est utilisée pour chauffer le tube de réaction à écoulement continu à la température souhaitée.

15. Procédé selon l'une des revendications précédentes, caractérisé en ce que l'acide succinique est en outre transformé en butanediol.

Figure 1

Figure 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5143834 A
- US 020190382328 A1
- JP 2006288361 A
- CN 104759244
- CN 101544758
- CN 201227587
- EP 4130532 A
- EP 24154108
- EP 4120532 A
- CN 113149822 B

**Non-patent literature cited in the description**

- **L. YANG et al.** *Carbohydrate Research*, 2011, vol. 346, 2304-2307
- **A. LORENTE et al.** *International Journal of Biological Macromolecules*, 2023, vol. 237, 124149